(19)

Europäisches
Patentamt
European
Patent Office
Office européen
des brevets

(11) **EP 4 718 462 A1**

(12) **EUROPEAN PATENT APPLICATION**

(43) Date of publication:
**01.04.2026 Bulletin 2026/14**

(21) Application number: **24203500.4**

(22) Date of filing: **30.09.2024**

(51) International Patent Classification (IPC):
***G16C 20/30*** (2019.01)  ***G16C 20/70*** (2019.01)
***G16C 60/00*** (2019.01)

(52) Cooperative Patent Classification (CPC):
**G16C 60/00;** G16C 20/30; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Bridgestone Europe NV/SA**
**1930 Zaventem (BE)**

(72) Inventors:
• **AGORETTI, Pasquale**
**00128 Roma (IT)**

• **MAGGI, Marco Andrea**
**00128 Roma (IT)**
• **MASSIMI, Lorenzo**
**00128 Roma (IT)**
• **CIAMBELLA, Jacopo**
**00185 Rome (IT)**
• **CALIFANO, Federico**
**00185 Rome (IT)**

(74) Representative: **Bardehle Pagenberg**
**Partnerschaft mbB**
**Patentanwälte Rechtsanwälte**
**Prinzregentenplatz 7**
**81675 München (DE)**

(54) **METHODS AND DEVICES FOR DETERMINING THE STRESS RESPONSE OF A VISCOELASTIC MATERIAL**

(57)    The present disclosure relates to determining the stress response of a viscoelastic material. More particularly, the present disclosure relates to computer-implemented methods for training a model to determine the stress response of viscoelastic material, methods for training a machine learning model to determine the viscosity of viscoelastic material, methods for generating augmented training data for determining the stress response of viscoelastic material, as well as to corresponding devices and computer programs. The methods, devices and computer programs as disclosed herein may be used for designing and manufacturing a tire.

**Figure 2**

**EP 4 718 462 A1**

**Description**

**Technical field**

The present disclosure relates to determining the stress response of a viscoelastic material. More particularly, the present disclosure relates to computer-implemented methods for training a model to determine the stress response of viscoelastic material, methods for training a machine learning model to determine the viscosity of viscoelastic material, methods for generating augmented training data for determining the stress response of viscoelastic material, as well as to corresponding devices and computer programs. The methods, devices and computer programs as disclosed herein may be used for designing and manufacturing a tire.

**Technical background**

Viscoelastic materials are widely used in various applications which is partly due to their unique ability to absorb and dissipate energy. Their versatile properties make viscoelastic materials indispensable in many modern technologies and everyday products. For example, in the automotive industry, viscoelastic materials are used to design tires and shock absorbers to dampen vibration and impacts. In the healthcare sector, viscoelastic materials are among others used in prosthetics and medical implants. Additionally, the viscoelastic materials are used in the design of shoes, where the viscoelastic properties help in reducing impact forces and preventing injuries.

Given the importance of viscoelastic materials in everyday life, there is a need to better understand the characteristics of viscoelastic materials, particularly the stress-strain relationship of viscoelastic materials. The stress-strain relationship describes how the material deforms under the application of force. While the behavior of viscoelastic materials under small and slow deformation is well-described in simple linear viscoelastic models, those models fail to capture the non-linear time-dependent effects that arise under large deformation, which is also referred to as finite deformation.

Conventional methods for determining the behavior of a viscoelastic material under small deformation include the assemblage of rheological elements. The fundamental concept of applying the assemblage of rheological elements to large and fast deformation is the assumption that, at each point, the deformation gradient can be multiplicatively decomposed into elastic and viscous components. The elastic part is usually represented as a spring, while the viscous part is usually represented as a dashpot (i.e., a damper). The elastic component describes the material's ability to return to its original shape after the applied force has been removed (storage of energy). In contrast, the viscous component describes the material's resistance to flow (dissipation or loss of energy).

Those conventional rheological models perform well regarding the determination of the elastic component. However, the conventional models struggle to accurately determine the viscous component of the model. Accordingly, conventional theoretical models are currently not able to capture the intricacies that are present in experimental data. A further disadvantage of purely theoretical models is their reliance on assumptions which may lead to inaccurate results that do not hold true in practical situations. Moreover, theoretical models are sometimes tailored to specific conditions which makes them not suitable for more general scenarios.

Thus, there is a need for determining more accurately the viscous properties of viscoelastic materials. A better understanding of these properties improves design and manufacture of products involving viscoelastic components. Against this background, an object of the present disclosure is to address one or more of the above-mentioned disadvantages of the conventional methods.

**Summary of the disclosure**

The above-mentioned objects and other objects, which become apparent from the following description, are solved by the subject-matter of the independent claims. Additional embodiments are the subject of the dependent claims.

A 1st embodiment of the disclosure is directed to a computer-implemented method for training a machine learning model to determine the viscosity of viscoelastic material, the method comprising: obtaining training data, wherein the training data is associated with stress information, strain information and viscosity information of a viscoelastic material; training the machine learning model based on the training data to enable the machine learning model to determine the viscosity of the viscoelastic material.

Obtaining training data, wherein the training data is associated with stress information, strain information and viscosity information of a viscoelastic material may have the advantage of enabling subsequent training of a machine learning model. Moreover, the training data being associated with stress information, strain information and viscosity information may reflect the desired input and output parameters of the machine learning model. Training the machine learning model based on the training data to enable the machine learning model to determine the viscosity of the viscoelastic material may have the advantage of calibrating the machine learning model to the training data.

According to a 2nd embodiment, obtaining training data comprises: generating training data using one or more constitutive models.

Generating training data using one or more constitutive models may have the advantage of calibrating the machine learning model. More specifically, using one or more constitutive models to generate training data may lead the machine learning model to mimic the viscosity function of the one or more constitutive models. The calibration and/or mimicking of the viscosity function that may result from generating training data using one or more constitutive models may improve the predictive capabilities of the machine learning model.

According to a 3rd embodiment, at least one of the one or more constitutive models accounts for non-linear dependencies between strain and stress.

Non-linear dependencies between strain and stress may be described as dependencies between an applied strain and a resulting stress that cannot be represented through a simple linear relationship. Non-linear dependencies may reflect complex behaviors such as deformation-enhanced shear thinning. Deformation-enhanced shear thinning may be described as a decrease in a material's viscosity due to an increase in deformation rate. In other words, when subjected to a fast deformation, the material may transition from a rather solid state to a rather fluid state. This phenomenon may also be referred to as the Payne effect in filled elastomers. At least one of the one or more constitutive models accounting for non-linear dependencies between strain and stress or preferably deformation-enhanced shear thinning, may further enhance the advantages mentioned with regards to the 2nd embodiment. In other words, it may improve calibration of the neural network and thus improve the predictive capabilities of the neural network.

According to a 4th embodiment, the output of the final layer of the machine learning model is positive to obtain a thermodynamically consistent output.

Having a positive output of the final layer of the machine learning model leads to a thermodynamically consistent output. The thermodynamically consistent output ensures that the physical process modeled by the machine learning model exhibits positive dissipation, thereby conforming to the fundamental principles of thermodynamics.

According to a 5th embodiment, the thermodynamically consistent output is obtained by: squaring the one or more weights of the final layer of the machine learning model; squaring the one or more biases of the final layer of the machine learning model; and ensuring that the one or more activations of the penultimate layer are positive.

Squaring the one or more weights of the final layer of the machine learning model may have the advantage of resulting in a positive value for each weight. For example, if a weight has been negative, the squaring would lead to the negative weight value becoming a positive weight value. Squaring the one or more biases of the final layer of the machine learning model may also have the advantage of resulting in a positive value for each bias. Ensuring that the processing of the one or more activations of the penultimate layer leads to a positive result may contribute to the thermodynamic consistency of the final output of the machine learning model. Moreover, the squaring and the ensuring may further enhance the advantages discussed with regards to the 4th embodiment. In other words, the steps may lead to a thermodynamically consistent output which may improve the predictive capabilities of the machine learning model.

A 6th embodiment of the disclosure is directed to a computer-implemented method for generating augmented training data for training a model to determine the stress response of viscoelastic material, the method comprising: interpolating, based on a first plurality of storage and loss modulus information of viscoelastic material, a second plurality of storage and loss modulus information; determining, based on the interpolating, augmented training data values and augmented training data labels.

Generating augmented training data for training a model to determine the stress response of viscoelastic material, in particular based on the interpolating, may have the advantage of effectively increasing the size of the training data set. An increased size of the training data set, comprising reliable additional data, may improve the training procedure of the machine learning model. An increased size of the training data set may further improve the predictive capabilities of the machine learning model.

Interpolating, based on a first plurality of storage and loss modulus information of viscoelastic material, a second plurality of storage and loss modulus information may enable the creating of meaningful training data points. Moreover, interpolation may be a less complex manner to increase the number of data points and may thus be computationally efficient and save computational resources. Moreover, interpolating may not only increase the number of training data points but may also enhance the resolution by generating intermediate values.

Determining, based on the interpolating, augmented training data values and augmented training data labels may enable the generation of augmented training data based on existing storage and loss modulus information. This may be advantageous since it may enable training the machine learning model using more training data without having to collect the training data. This may reduce the need for time and resource intensive experimental data collection.

According to a 7th embodiment, the augmented training data values comprise strain history information; and/or the augmented training data labels comprise stress history information.

The augmented training data values comprising strain information may improve the functionality of the machine learning model. More specifically, the training data values, which may define the input of the machine learning model, comprising strain history information may enable the determination of a stress response based on strain information. This may be advantageous in scenarios where strain information is essential for accurate stress computation in finite element analysis.

The augmented training data labels comprising stress information may enable the model to determine the stress response of viscoelastic material. More specifically, the training data labels, which may define the output of the machine learning model, comprising stress information may be advantageous for the determination of the stress response. Moreover, stress information may provide further relevant information about the behavior of the viscoelastic material that may be valuable for further processing.

According to an 8th embodiment, the plurality of storage and loss modulus information is experimental information, wherein the experimental information is preferably obtained using dynamic mechanical analysis, DMA.

The plurality of storage and loss modulus information being experimental information may have the advantage of basing the augmentation of the training data on real-world information. More specifically, experimental data may capture intricate behavior patterns of the material that could not have been captured using, for example theoretical models. Accordingly, experimental data may reveal unexpected patterns or anomalies that play an important role in real-life applications. This may in turn increase the predictive capabilities of the trained machine learning model.

Obtaining the experimental information using dynamic mechanical analysis may have the advantage of providing comprehensive information about the material's characteristics. Using dynamic mechanical analysis may also provide the advantage of standardization and comparability. In other words, dynamic mechanical analysis may be executed in such a manner that facilitates comparing the behavior of different materials.

A 9th embodiment of the disclosure is directed to computer-implemented method for training a model to determine the stress response of viscoelastic material, the method comprising: obtaining augmented training data according to the method of any one of embodiments 6 to 8; training the model based on the augmented training data, to enable the model to determine the stress response of viscoelastic material; and wherein the model comprises a machine learning model trained according to the method of any one of embodiments 1 to 5.

Obtaining augmented training data according to the method of any one of embodiments 6 to 8 may have all the advantages discussed with regards to the respective embodiments.

Training the model based on the augmented training data, to enable the model to determine the stress response of viscoelastic material may have the advantage of using a training data set with an increased number of samples. This may improve the training of the model and further improve the predictive capabilities of the model.

The model comprising a machine learning model trained according to the method of any one of embodiments 1 to 5 may have all the advantages discussed with regards to the respective embodiments. This may further enable indirectly training the machine learning model. More specifically, the structure of the model may enable training the machine learning model without requiring training data that corresponds to the input and/or output of the machine learning model. For example, a stress model (input = stress and strain, output = total stress) may enable training of a viscosity model (input = stress and strain, output = viscosity) without requiring information about the viscosity.

According to a 10th embodiment, the model further comprises: one or more components configured to reflect the elastic behavior of the viscoelastic material.

The model further comprises one or more components configured to reflect the elastic behavior of the viscoelastic material may have the advantage of accurately reflecting the relationship between the output of the machine learning model and the output of the model. In other words, the relationship between the viscosity of the viscoelastic material which is provided by the machine learning model and the total stress response which is provided by the output of the model. Thus, the incorporation of one or more components reflecting the elastic behavior may improve the predictive capabilities of the model.

According to an 11th embodiment, wherein training further comprises using a weighted loss function.

Using a weighted loss function may have the advantage of improving the training process and thus the predictive capabilities of the model during future use. More specifically, a weighted loss function may promote a balanced learning process across various experimental conditions. For example, the importance of a specific sample or type of sample of the training data set may be increased or decreased depending on the given weight.

A 12th embodiment of the disclosure is directed to a computer-implemented method for determining the stress response of viscoelastic material: inputting stress and strain information associated with a viscoelastic material into a model trained according to the method of any one of embodiments 9 to 11; and determining, using the model, the stress response of the viscoelastic material.

Inputting stress and strain information associated with a viscoelastic material into a model trained according to the method of embodiments 9 or 10 may have all the advantages discussed with regards to the respective embodiments. Determining, using the model, the stress response of the viscoelastic material may have the advantage of an improved accuracy of the determined stress response.

According to a 13th embodiment, the determined stress response of the viscoelastic material is used for designing and manufacturing a tire.

Using the determined stress response of the viscoelastic material for designing and manufacturing a tire may have the advantage of improving the tire. More specifically, an improved understanding of the material's stress response may allow for an improvement of the tire design parameters such as tread patterns and material composition which may lead to a better performance of the tire. Thus, reliability and safety of the tire may be improved. Moreover, as part of the design process, the determined stress response may be compared to the stress response of other materials. This may enable an improved process for selecting the most suitable material for the manufacturing of the tire. Using the determined stress response for designing and manufacturing a tire may also reduce the requirement for physical prototypes. This may reduce the number of iterations that are required to arrive at a final design of the tire and may also reduce waste. Using the determined stress response for designing and manufacturing a tire may further increase customization and innovation since the testing of new designs is less complex and less resource intensive overall.

A 14th embodiment of the disclosure is directed to a device or system comprising means for performing the method of any one of embodiments 1 to 13.

A device or system comprising means for performing the method of any one of embodiments 1 to 13 may have, but may not be limited to, all of the advantages mentioned with regards to embodiments 1 to 13.

A 15th embodiment of the disclosure is directed to a computer program comprising instructions which, when executed by a computer, cause the computer to carry out the method of any one of embodiments 1 to 13.

A computer program comprising instructions which, when executed by a computer, cause the computer to carry out the method of any one of embodiments 1 to 13 may have, but may not be limited to, all of the advantages mentioned with regards to embodiments 1 to 13.

**Brief description of the figures**

Various aspects of the present disclosure are described in more detail in the following by reference to the accompanying figures without the present disclosure being limited to the embodiments of these figures.

Fig. 1 illustrates the lack of performance in determining a loss modulus of a viscoelastic material using conventional methods;

Fig. 2 illustrates a comparison between a conventional framework (left) and a framework which incorporates a viscosity machine learning model according to an embodiment of the present disclosure (right);

Fig. 3 illustrates a flow chart representing the training process and a possible use case of a machine learning model according to an embodiment of the present disclosure;

Fig. 4 illustrates an exemplary version of a viscosity machine learning model according to an embodiment of the present disclosure;

Fig. 5 illustrates the interpolation of a data augmentation process according to an embodiment of the present disclosure;

Fig. 6 illustrates the performance difference between conventional methods and the stress machine learning model according to an embodiment of the present disclosure;

Fig. 7 further illustrates the performance difference between conventional methods and the stress machine learning model according to an embodiment of the present disclosure;

Fig. 8 illustrates an exemplary process for designing a tire according to an embodiment of the present disclosure.

**Detailed description of embodiments**

In the following, the embodiments are described with reference to the accompanying figures in more detail. However, the present disclosure can also be used in other embodiments not explicitly disclosed hereafter. As detailed below, the embodiments are compatible with each other, and individual features of one embodiment may also be applied to another embodiment.

Throughout the figures and description, the same reference numerals refer to the same elements, unless stated otherwise. The figures may not be drawn to scale, and the relative size, proportions, and depiction of elements in the figures may be exaggerated for the purpose of clarity, illustration, and convenience. The figures do not limit the scope of the claims but merely support the understanding of the embodiments.

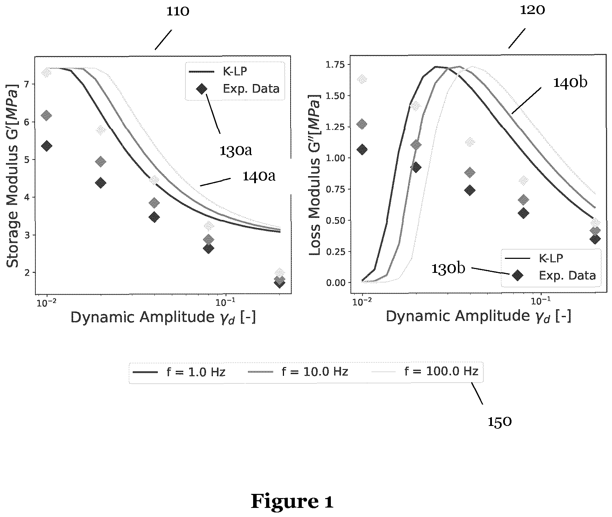

**Figure 1** compares the performance of the Kumar Lopez-Pamies (K-LP) model with the performance of experimental data with regards to determining the elasticity and the viscosity of a viscoelastic material. This comparison is shown via a storage modulus (G') graph 110 which describes the elastic properties of the viscoelastic material and a loss modulus (G") graph 120 which describes the disspative properties of the viscoelastic material. Both graphs 110, 120 show the applied sinusoidal strain in terms of the dynamic amplitude which may describe the amplitude of the applied sinusoidal strain on the x-axis and the storage modulus and loss modulus, respectively, on the y-axis.

The storage modulus graph 110 shows five experimental data points 130a for each frequency of the applied sinusoidal strain (1 Hz, 10 Hz, 100 Hz) 150. For a frequency of 100 Hz, the data points start at around 7.3 MPa and decrease to around 4.4 MPa and 2.0 MPa. A similar decreasing trend is measured for a frequency of 10 Hz (i.e., 6.2 MPa, 3.8MPa and 1.8MPa) and for a frequency of 1 Hz (5.3 MPa, 3.5 MPa and 1.7 MPa). It is important to note that the storage modulus values that were derived using the K-LP model follow the trajectory of the experimental data points as shown by the K-LP storage modulus curves 140a.

The loss modulus graph 120 also shows five experimental data points 130b for each frequency of the applied sinusoidal strain (1 Hz, 10 Hz, 100 Hz). For a frequency, of 100 Hz, the data points start at around 1.65 MPa and decrease to around 1.2 Ma, and 0.5 MPa. The same trend is measured for a frequency of 10 Hz (i.e., a decrease from 1.27 MPa to 0.49 MPa) and for a frequency of 1 Hz (i.e., a decrease from 1.06 MPa to 0.4 MPa). However, in contrast to the storage modulus, the loss modulus values derived by the K-LP model and shown in the loss graph 120 by the K-LP loss modulus curves 140b, do not closely follow the trajectory of the experimental data points 130b. While the experimental data monotonically decrease for all frequencies, the curves 140b have a non-monotonic behaviour and first increase and then decrease again with a significant difference at low dynamic amplitudes.

The present disclosure may have the advantage of improving the determination of the loss modulus (G") of viscoelastic material. Note that the loss modulus may be derived from the stress information that may be determined by the stress model that is suggested in the present application.

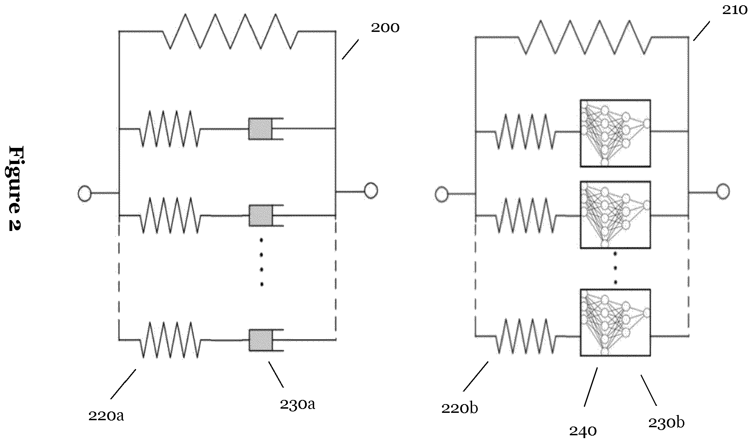

**Figure 2** shows a schematic representation of determining the behavior of a viscoelastic material as it is currently performed (i.e., state-of-the-art model 200) and as it is suggested in the present disclosure (i.e., suggested stress model) 210.

Both models comprise a part that describes the elastic behavior 220a, 220b of the viscoelastic material. Conventional methods perform well in this task. The suggested stress model may use conventional methods for the determination of the elastic component of the viscoelastic material.

Moreover, both models comprise a part that describes the inelastic behavior 230a, 230b of the viscoelastic material. As described with regards to Figure 1, conventional methods do not perform particularly well concerning the determination of the inelastic component of the viscoelastic material.

The present disclosure thus introduces a stress model 210, which may comprise a viscosity machine learning model 240. The viscosity machine learning model 240 may be pre-trained to determine the viscosity based on stress information and strain information and one or more constitutive models. Note that the stress information may be elastic stress information. Directly training the viscosity machine learning model 240 has proven to be difficult due to the lack of training data, in particular information about the viscosity. To circumvent this problem, the viscosity machine learning model 240 is indirectly trained via the stress machine learning model 210. In contrast to the viscosity model, which takes stress and strain as an input and provides viscosity as an output, the stress model takes stress and strain as an input and provides total stress as an output. Note that the stress may be elastic stress. Thus, the stress model may not require viscosity information for training. Accordingly, the weights and biases that are adjusted when training the stress model may be the weights and biases of the viscosity model.

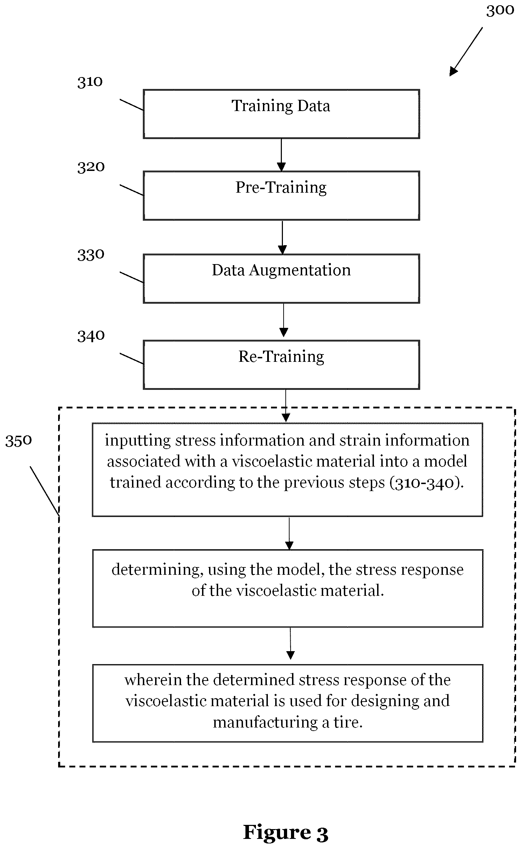

**Figure 3** uses a flow diagram 300 to schematically illustrate the various steps 310, 320, 330, 340 involved in the creation of a machine learning model that determines the inelastic component of a viscoelastic material. Note that some of the illustrated steps may also be performed in a different order, for example, the data augmentation 330 may take place before or after the pre-training phase 320.

The **first step 310** may comprise obtaining adequate training data. Obtaining adequate training data may further comprise obtaining training data from one or more established constitutive models such as the Kumar Lopez-Pamier model and/or the Strain Hardening Power Law (SHPL) model. Table 1 provides an overview of possible constitutive models that may be used for the obtaining of training data.

| Constitutive Model | Viscosity $\eta_D$ |
|---|---|
| Reese & Govindjee | Constant |
| Bergström & Boyce | $J_2^{1-m}/A(\lambda^v - 1 + \xi)^C$ with $\lambda^v = \sqrt{J_1/3}$ |
| Kumar & Lopez-Pamies | $\frac{1}{2}\left(\eta_\infty + \frac{\eta_0 - \eta_\infty + K_1\left(J_1^{\beta_1} - 3^{\beta_1}\right)}{1 + (K_2 J_2^2)^{\beta_2}}\right)$ |

(continued)

| Constitutive Model | Viscosity $\eta_D$ | | |
|---|---|---|---|
| Strain Hardening Power Law | $\dot{\varepsilon}^{cr} = (A J_2^n [(m+1) \bar{\varepsilon}^{cr}]^m)^{1/(m+1)}$ | with | $(4/3)(c/\Gamma)J_2^g$ |
| Drozdov and Pom-Pom model | | $c/\sqrt{2} \parallel \mathbf{D} \parallel$ | |
| Rubin & Papes | $J_2/\dot{\varepsilon}^{cr}$ | | |

Note that Table 1 merely provides a suggestion, and it is possible to use different models for obtaining the training data. A constitutive model that may be particularly useful in obtaining training data is the Kumar Lopez-Pamies model. This may be due to the simplicity of the model and the model's ability to capture shear-thinning, with a minimal number of parameters. The inclusion of shear-thinning may improve the predictive capabilities of the model.

The one or more constitutive models may be calibrated using experimental data. The experimental data may be obtained through experiments which comprise dynamic mechanical analysis (DMA). The one or more constitutive models may further be calibrated using an optimization algorithm. The optimization algorithm may for example be a particle swarm optimization algorithm, a genetic algorithm and/or a differential evolution algorithm.

The training data that is obtained in the first step 310 is associated with stress information, strain information and viscosity information of a viscoelastic material. In particular, the training data may comprise as an input stress information, strain information (i.e., information about the displacement history applied to a viscoelastic material) and as an output viscosity information (i.e., information about the viscous behavior of the material).

The **second step 320** may comprise training - also referred to as "pre-training" - a viscosity machine learning model based on the obtained training data to enable the machine learning model to determine the viscosity of the viscoelastic material. The viscosity machine learning model may be a neural network. The viscosity machine learning model may further comprise one or more hidden layers and/or an input layer and/or an output layer.

The obtained training data may be normalized preceding the pre-training of the viscosity machine learning model. This may improve the model's ability to effectively learn and generalize from the training data. A mean squared error (MSE) loss function, which quantifies the difference between the training data (e.g., obtained through K-LP model) and the calculated data (inference from the machine learning model) may be used during pre-training. The training data may also be split into training, test and validation data sets. This may improve the training process.

The viscosity machine learning model may further be designed to prevent thermodynamic inconsistencies. Thermodynamic inconsistency may describe a - situation in which a model violates fundamental principles of thermodynamics. Thermodynamic inconsistencies may be prevented by ensuring a positive output of the final layer of the machine learning model. More specifically, various operations such as the applications of exponentials to the weights and biases and different activation function of the penultimate layer may be used to ensure a positive output of the final layer. Even more specifically, the thermodynamic inconsistencies prevention design may comprise squaring the one or more weights of a layer the viscosity machine learning model, squaring the one or more biases of the layer of the viscosity machine learning model and ensuring that the processing of the one or more activations of the penultimate layer leads to a positive result. The layer may be the final layer of the viscosity machine learning model.

Note that the weights and biases may make up a second part of the equation and that the processing of the activation of the previous layer may make up a first part of the equation. Ensuring that the output remains strictly positive may also ensure thermodynamic consistency. Thermodynamic consistency may subsequently improve the accuracy of the results of the viscosity machine learning model.

The **third step 330** may comprise the augmentation of the obtained training data to obtain augmented training data. This augmented training data may further be used to indirectly re-train the viscosity model in the fourth step 440. As mentioned previously, the augmentation of the training data may also occur before the pre-training phase 320.

Data augmentation 330 may start with performing experiments to collect data about a materials storage and loss modulus. Those experiments may comprise dynamic material analysis. Note that the experiments that are performed to calibrate the one or more constitutive models in step 310 may also yield the storage and loss modulus information that is required for data augmentation.

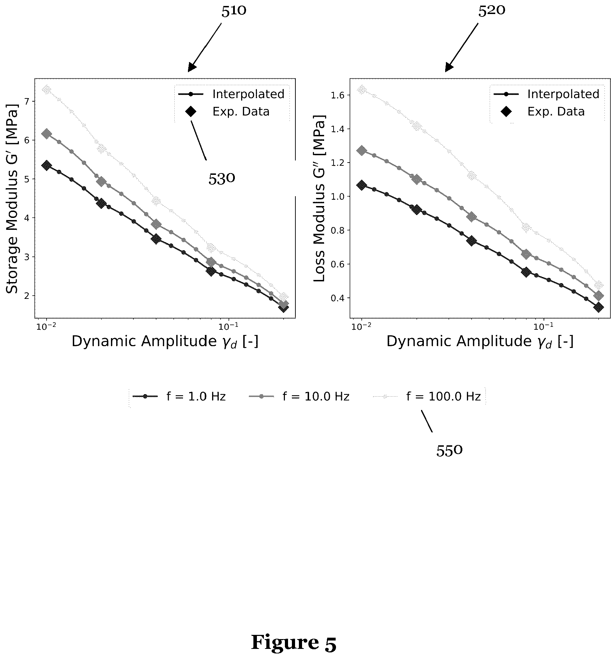

As a subsequent step of the data augmentation process 330, a plurality of storage and loss modulus values are interpolated based on the experimental storage and loss modulus values. The interpolated storage and loss modulus values may then be used to calculate training data values (i.e., stress and strain information) and training data labels (i.e., total stress information). This may have the advantage of increasing the size of the training data which may improve the performance of the model. Using augmented data may also have the advantage of saving resources since augmented data replaces experimental data. Note that the data augmentation process is described in more detail with regards to Figure 5.

The **fourth step 340** may comprise the training of the stress model which may also be referred to as the indirect re-training of the pre-trained viscosity machine learning model. The re-training may be performed using training data that is augmented according to the augmentation process described herein. A weighted mean squared error loss function may be used during the re-training process. A weighted loss function works by assigning a weight to each sample of the training data set. This may be advantageous since it enables the prioritization of specific samples. Moreover, weighting each sample may allow for fine tuning of the training process and thus improving the training process.

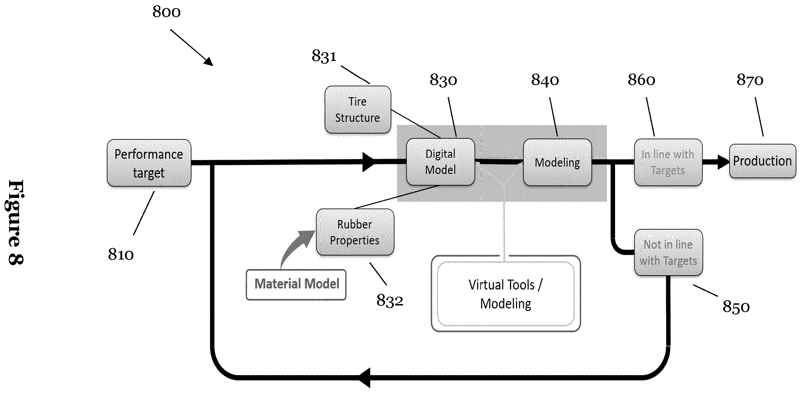

The **fifth step 350** further describes the use of the trained stress machine learning model. It may comprise inputting stress information and strain information associated with a viscoelastic material into a model trained according to the method of any one of embodiments 9 to 11. It may further comprise, determining, using the model, the stress response of the viscoelastic material. Finally, the stress response may be used for designing and manufacturing a tire. Note that the designing and manufacturing is described in more detail with regards to Figure 8.

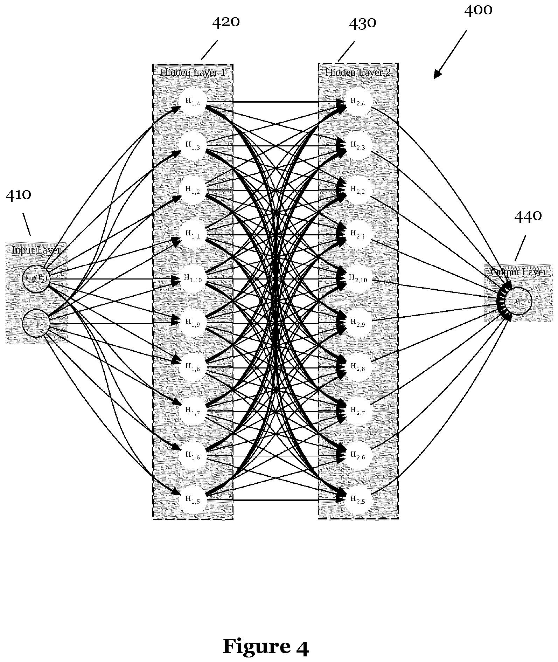

**Figure 4** shows an exemplary version of the viscosity machine learning model 400 which may be part of the stress machine learning model. The shown viscosity model is a simple feed forward neural network (FNN) which describes a neural network that processes input data in a single direction from input to output. In contrast to recurrent neural networks (RNN), FNNs do not contain any loops. An advantage of this architecture may be its simplicity which may also save computational resources. The exemplary network follows a 2-10-10-1 architecture, meaning that there are 2 neurons in the input layer 410, 10 neurons in the first hidden layer 420, 10 neurons in the second hidden layer 430 and a single neuron in the output layer 440. The input layer neurons are denoted as $J_1$ and log $(J_2)$. $J_1$ may describe the first invariant of the viscous strain tensor. Log($J_2$) may refer to the log value of the norm of the deviatoric stress tensor. The labels of the neurons present in the hidden layer (i.e., $H_{1,1}$, $H_{2,1}$) can be seen as an identification number of the neuron. The first number indicates the layer of the neuron, and the second number indicates the neuron in the specific layer. Note that the exemplary network is a fully connected neural network, meaning that each neuron in one layer is connected to each neuron in the subsequent layer. The final layer is indicated as $\eta$ describes the viscosity as the output of the viscosity machine learning model.

**Figure 5** illustrates the interpolation which may be part of the data augmentation. More specifically, Figure 5 shows to interpolation graphs 510, 520. A first interpolation graph 510 relates to the storage modulus (G') 510 and a second interpolation graph 520 relates to the loss modulus (G"). The x-axis of each graph displays the dynamic amplitude. The graphs show the trajectory of the storage modulus and loss modulus with regards to dynamic amplitude (i.e., the amplitude of the applied sinusoidal strain).

It can be seen that the storage modulus curves 510 and the loss modulus curves 520 each contain five experimental data points 530 for each frequency. Note that the curves 510, 520 are not continuous curves but rather discrete data points that appear as a curve. A first step of the data augmentation process, which may be used to increases the size of the training data set, may be the interpolation of storage modulus values and/or loss modulus values. Those interpolated values are represented by the round data points in the interpolation graph. For example, between the second experimental data point and the third experimental data point of the storage modulus curves 510 of each frequency, there are multiple interpolated data points. The interpolated values may be evenly distributed, for example one value may be interpolated per unit of dynamic amplitude. Techniques such as spline interpolation and/or polynomial interpolation may be used for interpolating the storage and loss modulus values.

The aim of the data augmentation process may be to increase the size of the training data set. Accordingly, based on the interpolated storage and loss modulus information, training data values (i.e., input) in form of stress information and strain information and training data labels (i.e., output) in form of total stress information may be derived. This may be performed using the following equations, wherein y(t) represents the strain history information, $T(t)$ represents the stress history information, $y_0$ represents the static strain, $y_d$ represent the dynamic amplitude, $\mu_\infty$ represents the modulus of a long-term spring, wherein the modulus may be obtained from the constitutive model, $\omega$ and t represent the angular frequency and time, respectively.

$$y(t) = y_0 + y_d sin(\omega t)$$

$$T(t) = \mu_\infty \gamma_0 + y_d G' \sin(\omega t) + y_d G'' \cos(\omega t)$$

The result of the data augmentation process may be a training set that comprises an increased number of samples. Each sample may comprise of stress information and strain information as an input and total stress information as an output.

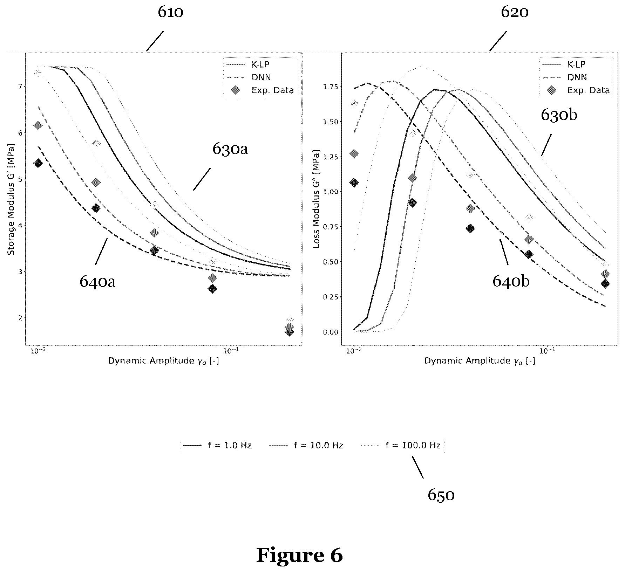

**Figure 6** compares the performance of the machine learning model, the K-LP model and the experimental data with regards to determining the storage modulus and the loss modulus of a viscoelastic material. More specifically, Figure 6 depicts the same storage modulus graph 610 and loss modulus graph 620 that are described in Figure 2. However, Figure 6 also includes storage and loss value determinations of the stress machine learning model as introduced in the present application (denoted here as DNN) 640a, 640b.

The storage modulus graph 610 shows that both, the values determined using the K-LP model (K-LP curves 630a) and the values determined using the DNN model (DNN curves 640a), closely follow the experimental data points. It is noticeable that the DNN curves 640a are better at matching the initial four experimental data points compared to the K-LP curves 630a. Both sets of curves 630a, 640a struggle to match the last experimental data points. The loss modulus graph 620 shows that the DNN curves 640b accurately match the final experimental data points and performs well for the data points at the third and the fourth dynamic amplitudes. In contrast, the K-LP curves 630b perform reasonably well in matching the data points of the second dynamic amplitudes at 1 and 10 Hz (i.e., the second experimental data points), however, the K-LP curves 630b grossly mismatches the remaining experimental data points.

The above-described performance of the K-LP model and the DNN model may also be shown using the mean relative error (MRE). The DNN model has an MRE of 0.17 for the storage modulus value and the K-LP model has an MRE of 0.36 for the storage loss value (for readability the final two numbers have been omitted).

This means that, using the DNN model, a determined storage modulus value, on average, deviates by 17% from the actual storage modulus value. Using the K-LP model, this value is 36%. Accordingly, the DNN model shows a better performance in determining a storage modulus value compared to the K-LP model. The performance difference becomes even more pronounced when comparing the results for determining the loss modulus value. More specifically, using the DNN model, a determined loss modulus value, on average, deviates by 35% from the actual value. In contrast, using the K-LP model, a determined loss modulus value, on average, deviates by 72% from the actual value. Thus, the DNN model may significantly outperform the K-LP model regarding the determination of a loss modulus value of a viscoelastic material.

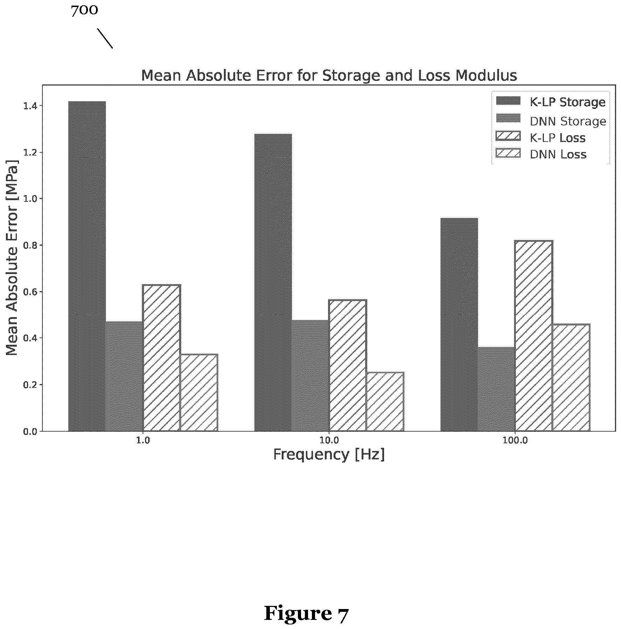

Figure 7 depicts a performance graph 700 that shows the mean absolute error (MPa) of the determined storage and loss moduli using the K-LP model and the DNN model) for three different frequencies (i.e., 1 Hz, 10 Hz, 100 Hz).

Using a 1 Hz frequency, the DNN model is on average able to determine the storage modulus and the loss modulus at a lower MPa error then the K-LP model. More specifically, the DNN model achived an error of around 0.62 MPa and 0.38 MPa for the storage modulus and loss modulus, respectively. In contrast, the K-LP model determined the storage modulus with an error of 1.41 MPa and the loss moulus with an error of 0.44 MPa. Similar values can be observed with regards to a frequency of 10 Hz. It is notable that the performance of the DNN model is signifcanlty better than that of the K-LP model regarding the determination of the storage modulus. Using a 10 Hz frequency, the DNN model achieves a slightly lower error than the K-LP model (0.80 MPa as compared to 0.90 MPa) regarding the determination of the storage modulus. Regarding the loss modulus, the DNN model achvies an error of 0.43 MPa, while the K-LP model achives an error of 0.38 MPa. The results further illustrate the improvement in the determination of the storage modulus and the loss modulus resulting from the present disclosure.

**Figure 8** demonstrates the process of designing a tire 800 as an exemplary use case for the stress machine learning model. A respective design process may start with defining target parameters for the performance of the tire 810, end with the actual production of the tire 870 and contain several design iterations 820.

As shown in Figure 8, the definition of the target parameters 810 may comprise the definition of a specific performance regarding the wear of the tire or characteristics such as the tire's ability to maintain grip on wet or dry surfaces or to save energy while rolling. Once the target parameters are defined, the first design iteration may start. Each design iteration may comprise the construction of a digital representation of a proposed tire 830. Constructing the digital representation of the proposed tire requires information about the tire structure 831 and information about the properties of the rubber (i.e., the viscoelastic material) 832. In the subsequent step 840, the digital representation of the proposed tire is used to investigate the behavior of the proposed tire design under various circumstances. Note that this step may eliminate the production of a first prototype and thus save resources. The results of the investigation are then compared to the previously defined target parameters. In case that the results are not in line with the defined target parameters 850, a further design iteration may be triggered. In case that the results are in line with the target parameters 860, the design may be approved for production 870.

Embodiments of the present disclosure may be realized in any of various forms, e.g., in software. For example, in some embodiments, the present disclosure may be realized as a computer-implemented method, a computer-readable memory medium, or a computer system.

In some embodiments, a non-transitory computer-readable memory medium may be configured so that it stores program instructions and/or data, where the program instructions, if executed by a computer system, cause the computer system to perform a method, e.g., any of the method embodiments described herein, or, any combination of the method embodiments described herein, or, any subset of any of the method embodiments described herein, or, any combination of such subsets.

In some embodiments, a computing device may be configured to include a processor (or a set of processors) and a memory medium, where the memory medium stores program instructions, where the processor is configured to read and execute the program instructions from the memory medium, where the program instructions are executable to implement any of the various method embodiments described herein (or, any combination of the method embodiments described herein, or, any subset of any of the method embodiments described herein, or, any combination of such subsets). The device may be realized in any of various forms.

Although specific embodiments have been described above, these embodiments are not intended to limit the scope of the present disclosure, even where only a single embodiment is described with respect to a particular feature. Examples of features provided in the disclosure are intended to be illustrative rather than restrictive unless stated otherwise. The above description is intended to cover such alternatives, modifications, and equivalents as would be apparent to a person skilled in the art having the benefit of this disclosure.

The scope of the present disclosure includes any feature or combination of features disclosed herein (either explicitly or implicitly), or any generalization thereof, whether or not it mitigates any or all of the problems addressed herein. In particular, with reference to the appended claims, features from dependent claims may be combined with those of the independent claims and features from respective independent claims may be combined in any appropriate manner and not merely in the specific combinations enumerated in the appended claims.

**List of reference signs**

| | |
|---|---|
| 110, 610 | storage modulus graph |
| 120, 620 | loss modulus graph |
| 130a, 130b | experimental data points |
| 140a, 140b | K-LP curves |
| 150, 550, 650 | frequency of the dynamic amplitude |
| 200 | conventional model |
| 210 | stress machine learning model |
| 220a, 220b | elastic behavior |
| 230a, 230b | inelastic behavior |
| 240, 400 | viscosity machine learning model |
| 300 | flow diagram |
| 310 | obtaining training data |
| 320 | pre-training phase |
| 330 | data augmentation process |
| 340 | re-training phase |
| 350 | design & manufacturing phase |
| 400 | viscosity neural network |
| 410 | input layer |
| 420 | first hidden layer |
| 430 | second hidden layer |
| 440 | output layer |
| 510 | interpolation graph of the storage modulus curves |
| 520 | interpolation graph of the loss modulus curves |
| 530 | experimental data points |
| 630a, 630b | K-LP curves |
| 640a, 640b | DNN curve |
| 700 | performance graph |
| 800 | tire design process |
| 810 | target parameters |
| 830 | digital representation of proposed tire |
| 831 | tire structure |
| 832 | stress model |
| 840 | investigation of characteristics |
| 850 | not in line with defined target parameters |
| 860 | in line with defined target parameters |
| 870 | production |

**Claims**

1. A computer-implemented method for training a machine learning model to determine the viscosity of viscoelastic material, the method comprising:

obtaining training data, wherein the training data is associated with stress information, strain information and viscosity information of a viscoelastic material;

training the machine learning model based on the training data to enable the machine learning model to determine the viscosity of the viscoelastic material.

2. The method of claim 1, wherein obtaining training data comprises:

generating training data using one or more constitutive models.

3. The method of claim 2, wherein at least one of the one or more constitutive models accounts for non-linear dependencies between strain and stress.

4. The method of any one the preceding claims, wherein the output of the final layer of the machine learning model is positive to obtain a thermodynamically consistent output.

5. The method of claim 4, wherein the thermodynamically consistent output is obtained by:

squaring the one or more weights of the final layer of the machine learning model;

squaring the one or more biases of the final layer of the machine learning model; and

ensuring that the processing of the one or more activations of the penultimate layer lead to a positive result.

6. A computer-implemented method for generating augmented training data for training a model to determine the stress response of viscoelastic material, the method comprising:

interpolating, based on a first plurality of storage and loss modulus information of viscoelastic material, a second plurality of storage and loss modulus information;

determining, based on the interpolating, augmented training data values and augmented training data labels.

7. The method of claim 6, wherein the augmented training data values comprise strain history information; and/or wherein the augmented training data labels comprise stress history information.

8. The method of claim 6 or claim 7, wherein the plurality of storage and loss modulus information is experimental information, wherein the experimental information is preferably obtained using dynamic mechanical analysis, DMA.

9. A computer-implemented method for training a model to determine the stress response of viscoelastic material, the method comprising:

obtaining augmented training data according to the method of any one of claims 6 to 8;

training the model based on the augmented training data, to enable the model to determine the stress response of viscoelastic material; and

wherein the model comprises a machine learning model trained according to the method of any one of claims 1 to 5.

10. The method of claim 9, wherein the model further comprises:

one or more components configured to reflect the elastic behavior of the viscoelastic material.

11. The method of claim 9 or claim 10, wherein training further comprises using a weighted loss function.

12. A computer-implemented method for determining the stress response of viscoelastic material:

inputting stress information and strain information associated with a viscoelastic material into a model trained according to the method of any one of claims 9 to 11; and

determining, using the model, the stress response of the viscoelastic material.

13. The method of claim 12, wherein the determined stress response of the viscoelastic material is used for designing and manufacturing a tire.

14. A device or system comprising means for performing the method of any one of claims 1 to 13.

15. A computer program comprising instructions which, when executed by a computer, cause the computer to carry out the method of any one of claims 1 to 13.

**Figure 1**

**Figure 2**

300

310 ── Training Data

320 ── Pre-Training

330 ── Data Augmentation

340 ── Re-Training

350

inputting stress information and strain information associated with a viscoelastic material into a model trained according to the previous steps (310-340).

determining, using the model, the stress response of the viscoelastic material.

wherein the determined stress response of the viscoelastic material is used for designing and manufacturing a tire.

**Figure 3**

**Figure 4**

Figure 5

Figure 6

Figure 7

**Figure 8**

| | Europäisches Patentamt<br>European Patent Office<br>Office européen des brevets | **EUROPEAN SEARCH REPORT** | **Application Number**<br>EP 24 20 3500 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ABDOLAZIZI KIAN P. ET AL: "Viscoelastic constitutive artificial neural networks (vCANNs) - A framework for data-driven anisotropic nonlinear finite viscoelasticity",<br>JOURNAL OF COMPUTATIONAL PHYSICS.,<br>vol. 499, 1 February 2024 (2024-02-01),<br>page 112704, XP093252768,<br>GB<br>ISSN: 0021-9991, DOI:<br>10.1016/j.jcp.2023.112704 | 1-5,14,<br>15 | INV.<br>G16C20/30<br>G16C20/70<br>G16C60/00 |
| Y | * p. 1, Abstract<br>p. 7, Figure 2 and description thereof<br>p. 7, last paragraph<br>p. 8, penultimate paragraph<br>p. 9, 1 and 2<br>p. 12, Section 4.3 Viscolelastic modelling of VHB 4910 *<br>----- | 6-15 | |
| Y | CHENGYU GUAN ET AL: "A method of automatically obtaining master surfaces of resin matrix composites by time-temperature superposition",<br>POLYMER COMPOSITES, SOCIETY OF PLASTICS ENGINEERS, INC, US,<br>vol. 43, no. 9,<br>10 August 2022 (2022-08-10), pages 6389-6403, XP072706878,<br>ISSN: 0272-8397, DOI: 10.1002/PC.26951 | 6-15 | TECHNICAL FIELDS SEARCHED (IPC)<br><br>G16C |
| A | * p. 6389, Abstract<br>p. 6403, left column, 2 *<br>-----<br><br>-/-- | 1-5 | |

The present search report has been drawn up for all claims

| Place of search | Date of completion of the search | Examiner |
|---|---|---|
| Munich | 24 February 2025 | Mühlbauer, Max |

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 20 3500

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LINKA KEVIN ET AL: "Constitutive artificial neural networks: A fast and general approach to predictive data-driven constitutive modeling by deep learning", JOURNAL OF COMPUTATIONAL PHYSICS, LONDON, GB, vol. 429, 24 November 2020 (2020-11-24), XP086485968, ISSN: 0021-9991, DOI: 10.1016/J.JCP.2020.110010 [retrieved on 2020-11-24] * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 24 February 2025 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 2 of 2